## Europäisches Patentamt
## European Patent Office
## Office européen des brevets

(19)

(11) Veröffentlichungsnummer : **0 372 217 B1**

(12)
# EUROPÄISCHE PATENTSCHRIFT

(45) Veröffentlichungstag der Patentschrift :
03.05.95 Patentblatt 95/18

(51) Int. Cl.$^6$ : **G01N 33/543**

(21) Anmeldenummer : **89120156.8**

(22) Anmeldetag : **31.10.89**

(54) **Einschritt-Immuntest zur Bestimmung von antigen-spezifischen Antikörpern aller Immunglobulin-Klassen und dazu geeignetes Mittel.**

(30) Priorität : **05.11.88 DE 3837616**

(43) Veröffentlichungstag der Anmeldung :
**13.06.90 Patentblatt 90/24**

(45) Bekanntmachung des Hinweises auf die
Patenterteilung :
**03.05.95 Patentblatt 95/18**

(84) Benannte Vertragsstaaten :
**AT BE CH DE ES FR GB IT LI LU NL SE**

(56) Entgegenhaltungen :
**EP-A- 0 062 892**
**EP-A- 0 111 762**
**EP-A- 0 168 689**
**EP-A- 0 277 437**
**EP-A- 0 292 810**

(56) Entgegenhaltungen :
**WO-A-85/02258**
**WO-A-88/07680**
**US-A- 4 016 043**
**Derwent Publications Ltd., London, GB; Database WPIL, accession no. 85-227879, week 8537; & RD-256011 [ANONYMOUS] 10 August 1985**

(73) Patentinhaber : **BEHRINGWERKE Aktiengesellschaft**
**Postfach 1140**
**D-35001 Marburg (DE)**

(72) Erfinder : **Krupka, Udo, Dr.**
**Oberer Eichweg 29**
**D-3550 Marburg (DE)**
Erfinder : **Pauly, Hans Erwin, Dr.**
**Finkenstrasse 1**
**D-3563 Dautphetal (DE)**

## Beschreibung

Die Erfindung betrifft ein immunchemisches Verfahren zum Nachweis und zur Bestimmung von Antikörpern, die für ein bestimmtes Antigen spezifisch sind und die alle Immunglobulin-Klassen umfassen.

Immunglobuline sind Antikörper, die vom Immunsystem des Organismus gegen Fremdstoffe (Antigene, z.B. Proteine von Krankheitserregern, bakterielle Polysaccharide) gebildet werden, aber auch gegen körpereigene Serumproteine und Gewebeproteine.

Die Bestimmung von antigenspezifischen Immunglobulinen ist von besonderer Bedeutung für den Nachweis bestimmter Erkrankungen durch Parasiten, Bakterien oder Viren. Zur Bestimmung von Immunglobulinen sind eine Vielzahl immunologischer Methoden bekannt. Methoden zur physikalischen Trennung von Immunglobulinen nach Klassen, z.B. Immundiffusion, Immunelektrophorese oder Dichtegradienten-Zentrifugation, sind aufwendig, ungenau und störanfällig.

Antigenspezifische Immunglobuline können im sogenannten direkten Verfahren mit Immunoassay-Techniken bestimmt werden; dabei wird das Antigen an einen festen Träger gekoppelt, wobei eine reaktive Festphase erhalten wird. Die Körperflüssigkeit, welche das antigenspezifische Immunglobulin enthält, wird mit der Festphase zusammengebracht, wobei das antigenspezifische Immunglobulin an die Festphase gebunden wird, Festphase und flüssige Phase werden getrennt, die Festphase wird anschließend mit einem in Lösung befindlichen markierten Antigen, welches die gleiche Spezifität hat wie das Antigen der Festphase, zusammengebracht, Festphase und flüssige Phase werden wieder getrennt und die Markierung entweder an der Festphase oder in der flüssigen Phase bestimmt. Ein solches Verfahren ist in Beispiel III von USP 4,016,043 beschrieben.

Zur Markierung werden beispielsweise fluoreszierende und chromophore Stoffe oder radioaktive Isotope oder Enzyme verwendet.

Bei der genannten Methode ist es essentiell, nach Umsetzung (Inkubation) die die nachzuweisenden Antikörper enthaltenen Probe mit der Festphase und vor Umsetzung mit dem markierten Antigen, ungebundenes Material durch Waschung zu entfernen. Diese Verfahren werden deshalb als "Zweischritt-Verfahren" bezeichnet.

Dadurch erfordert ein Test mit vorgefertigter Festphase mindestens drei Reaktionsschritte, nämlich Zugabe

1. von Probe,
2. von markiertem Antigen und
3. den Reagenzien für die Nachweisreaktion,

wobei jeder Reaktionsschritt noch mindestens 2 Waschschritte erforderlich macht.

In der EP-A 0 169 689 wird ein Einstufen Doppel-Antigen Sandwichassay zur Bestimmung von antigenspezifischen Antikörpern beschrieben, wo die Antigene heterolog sein müssen.

In Derwent Abstract RD 256011 (Acc.No. 85-227879) wird Doppel-Antigen Sandwichassay zur Bestimmung von Interferon beschrieben, wobei die Festphasenbindung des Fängerantigens über Avidin/Biotin erfolgt.

In der EP-A 0 062 892 wird ein Einstufen Doppel-Antikörper Sandwichassay beschrieben.

Die Aufgabe bestand nun darin, den Test zu verkürzen. Überraschend wurde nun gefunden, daß dies möglich ist, indem die antigenhaltige Festphase, die Antikörper enthaltende Probe und markiertes Antigen zusammengebracht werden, ohne zwischen der Zugabe der Probe und der Zugabe des markierten Antigens zu waschen.

Gegenstand der Erfindung ist daher ein Verfahren gemäß Anspruch 1.

Als Trägermaterial für die Festphase sind Kunststoffe wie Polystyrol, Polyvinylchlorid, Polyamid oder andere Synthetische Polymere, natürliche Polymere wie Zelluloseacetat oder Nitrozellulose als auch Glas, insbesondere Glasfasern geeignet.

Die Träger können die Form von Kugeln, Stäben, Röhrchen und Mikrotestplatten haben. Flächenförmige Gebilde wie Streifenpapiere, Plättchen und Membranen sind ebenfalls geeignet. Die Oberfläche der Träger kann für wäßrige Lösungen sowohl durchlässig als auch undurchlässig sein.

Bevorzugte Träger sind Mikrotestplatten.

Eine für das erfindungsgemäße Verfahren geeignete Festphase wird hergestellt, indem eine Antigenpräparation irreversibel an einen Träger gebunden wird.

Der Ausdruck "Festphase" wird in der vorliegenden Schrift für den Träger mit daran gebundenen immunchemischen Reaktanten gebraucht.

Eine irreversible Bindung im Sinne der Erfindung liegt beispielsweise vor bei

1) einer adsorptiven Bindung, die nicht durch die in dem Verfahren verwendeten Mittel wie markierte Immunereagenzien, Verdünnungs- oder Pufferlösungen gespalten wird,

2) einer kovalenten direkten Bindung oder

3) einer kovalenten, durch einen chemisch bifunktionellen Spacer vermittelten Bindung.

Bevorzugt wird die kovalente Bindung im Falle der Verwendung von wasserdurchlässigen Trägern oder die adsorptive Bindung im Falle der Verwendung von wasserdurchlässigen als auch wasserundurchlässigen Trägern.

Besonders bevorzugt wird die direkte adsorptive Bindung von Antigenpräparationen an mit Gammastrahlen behandeltes Polystyrol als Träger.

Als Antigene für die Festphase sowie zur Herstellung markierter Antigene kommen klassisch gereinigte Proteine, synthetische Peptide oder gentechnisch hergestellte Proteine in Frage, deren Darstellung als Stand der Technik beschrieben ist.

Zum Herstellen der markierten Antigene werden im allgemeinen die gleichen Antigene verwendet wie die zur Bindung an den Träger. Die Markierung erfolgt nach Verfahren, die für die genannten Marker als Stand der Technik beschrieben sind.

Zur Markierung werden beispielsweise fluoreszierende und chromophore Stoffe oder radioaktive Isotope, Enzyme oder mit Immunkomponenten beladene Partikel wie Erythrozyten oder Latexpartikel verwendet.

Vorzugsweise wird ein Enzym verwendet.

Mit dem erfindungsgemäßen Mittel werden Antikörper nachgewiesen und bestimmt, gerichtet z. B. gegen Cytomegalie-Virus, Rubella, Herpes Simplex Virus, Varizella Zoster Virus, Masern und Mumps, Toxoplasma gondii, Treponemen, Hepatitis A- und B-Virus, Human Immunodeficiency Virus, Hepatitis B c- und e-Antigen, Chlamydia, Epstein-Barr-Virus, Parvovirus, Rotavirus sowie gegen Erreger, die in der Veterinär-Diagnostik von Bedeutung sind, beispielsweise die Erreger der Aujeszki-Krankheit, Rinderleukose oder der Brucellose.

Vorzugsweise wird das erfindungsgemäße Mittel zur Bestimmung von Antikörpern verwendet, die gegen Hepatitis B-core Protein, gegen Antigene von Hepatitis A-, Human Immunodeficiency- (HIV), Röteln- oder Cytomegalie-Virus oder Antigene von Treponema pallidum oder Toxoplasma gondii gerichtet sind.

Bevorzugt ist auch ein solches Mittel, das aus einem einzigen Element, in dem alle für das Verfahren erforderlichen Reagenzien in trockener Form enthalten sind, besteht.

Die Anwendungsmöglichkeiten der beschriebenen Erfindung eines Einschritt-Immuntests sind grundsätzlich identisch mit den Anwendungen der bereits vorbeschriebenen direkten und indirekten mehrstufigen Tests. Von diesen unterscheidet sich das vorliegende neue Verfahren in dreierlei Hinsicht vorteilhaft:

Durch die Simultan-Inkubation von analyt-haltiger Probe und markierten Immunreagenzien entfällt ein Inkubationsschritt und ein Waschvorgang, was eine erhebliche Vereinfachung der Testdurchführung zur Folge hat.

Wie aus den Beispielen ersichtlich wird, gestattet das Einschritt-Verfahren eine erhebliche Verkürzung der Gesamtdauer des Tests, was neben dem vordergründigen Vorteil einer praktikablen Handhabbarkeit des Verfahrens auch in der Klinik für den raschen Nachweis akuter Infektionen eine große Bedeutung besitzt.

Die folgenden Beispiele stellen Ausführungsformen der Erfindung dar, ohne sie jedoch darauf zu beschränken.

Beispiel 1

Bestimmung von Hepatitis B surface-Antigen-spezifischen Immunglobulinen in humanen Seren oder Plasmen.

A. Herstellung von Hepatitis B surface-Antigen (HBs)

HBs-Protein wurde aus Humanseren hergestellt, die hohe Konzentrationen von Hepatitis B-Antigenen des Subtyps adw oder ady aufwiesen. Zur Anwendung kamen Chromatographie- und Zentrifugations-Verfahren, die dem Stand der Technik entsprachen:

Gerlich et al., 1975, Developments in Biological Standardization 30, 78 - 87; Gerin et al., 1975 J. Immunol. 115, 100 - 105. Die Konzentration der so hergestellten Präparation an Hepatitis B surface-Antigen wurde gemäß der von Stamm et al., 1980, J. of Biological Standardization beschriebenen Methode quantifiziert.

B. Markierung des Hepatitis B-surface-Antigens (HBs) mit Meerrettich-Peroxidase (POD)

Die Markierung der HBs-Präparation aus Humanserum mit POD erfolgte nach Rekonstitution der Proteinlösung auf 4 mg/ml in phosphat-gepufferter Kochsalzlösung, pH 7.2 gemäß der Periodat-Technik nach Nakane et al., 1974, J.Histochem.Cytochem. 22, 1084-1090. Es werden dabei 20 mg POD in 1 ml phosphat-gepufferte Kochsalzlösung, pH 7.4 gelöst und nach Zugabe von 2,4-Dinitrofluorbenzol mit Natriummetaperjodat behandelt. Anschließend wurde an $^R$Biogel P 6, equilibriert mit 0.2 mol/l Natriumcarbonat/-hydrogencarbonat, pH 9.5, gelfiltriert und die Fraktion der POD, die Aldehydgruppen enthielt mit 4 mg/ml HBs reagieren lassen. Nach Zugabe von Natriumborhydrid wurde wieder gelfiltriert und die POD- und HBs-haltige Fraktion als POD-HBs Konjugat mit einem Proteingehalt von 1 mg/ml und einem POD:HBs Verhältnis von 1.5:1 bis 2.0:1 erhalten.

Dieser Fraktion wurden 20 mg/ml Rinderserumalbumin und 1 mg/ml Phenol zugesetzt. Zur weiteren Verdünnung wurde eine als Konjugatpuffer bezeichnete Lösung von 5 g/l hydrolysierter und mit Hexamethylendiisocyanat vernetzter Gelatine, einem als Polygeline bezeichneten Handelsprodukt der Behringwerke AG, und 50 mmol/l Tris, eingestellt mit HCl auf pH 7.4, verwandt.

Zur Ermittlung der Konjugatverdünnungen sowohl für das Beispiel D (Stand der Technik) als auch für das Beispiel E (erfindungsgemäßes Einschrittverfahren) wurden serielle Verdünnungen in Zweier- als auch Fünferstufen hergestellt, wobei alle Verdünnungen mit den in der Tabelle genannten Plasmen und der Negativ-kontrolle und jeweils alle mit verschiedenen Mengen an HBs beschichteten Vertiefungen der Microtestplatten gemäß den beiden Verfahren zusammengebracht wurden.

Es wurde gefunden, daß Konjugatverdünnungen von 1:50 und Vertiefungen, beschichtet mit Lösun-gen von 0.4 µg/ml HBs (siehe Beispiel C und D) im Zweischrittverfahren vergleichbare Ergebnisse brachten wie Konjugatverdünnungen von 1:20 und Vertiefungen, beschichtet mit Lösungen von 1.0 µg/ml HBs im Einschrittverfahren (siehe Beispiel C und E).

C. Beschichtung von Mikrotestplatten mit HBs

Mikrotestplatten, d. h. Immunoplatten II 96 F mit rundem Boden (Fa. Nunc, Roskilde, Dänemark, Art.-Nr. 262162) wurden pro Vertiefung mit 125 µl von Lösungen mit 1.6, 1.0, 0.8, 0.4, 0.2 und 0.1 µg/ml HBs-Antigen in phosphatgepufferter Kochsalzlösung, pH 7,5, mehrere Stunden bei Raumtemperatur inkubiert. Anschließend wurden die Platten leergesaugt, mit phosphatgepufferter Kochsalzlösung gewaschen, mittels Silikagel getrocknet und luft- und wasserdampfdicht verpackt.

D. Bestimmung von HBs-antigenspezifischen Antikörpern gemäß dem Stand der Technik im Zweischrittverfahren

a) Pro Vertiefung einer Mikrotestplatte beschichtet mit einer Lösung von 0.4 µg/ml HBs, wurden 200 µl der zu untersuchenden unverdünnten Serumprobe aufgetragen. Als Vergleich wurden 200 µl unver-dünntes, HBs-Antigen-Antikörper-freies Humanserum aufgetragen (Negativ-Kontrolle) sowie serielle Verdünnungen eines Antikörpers gegen HBs-Antigen enthaltendende Proben;

b) Die Testplatte wurde abgedeckt ung 16 bis 24 h bei 18 - 25 °C inkubiert;

c) Anschließend wurde der Inhalt abgesaugt und 3mal mit phosphat-gepufferter Kochsalzlösung, enthaltend 0,1 ml Tween[R] 20, gewaschen;

d) Nun wurde pro Vertiefung 100 µl Konjugatverdünnung von 1:50 zugegeben;

e) Die Testplatte wurde abgedeckt und für 1 h bei 40 °C inkubiert;

f) Nach erneutem dreimaligem Waschen mit phosphatgepufferter Kochsalzlösung, pH 7,5, wurden pro Vertiefung 100 µl Chromogen-Lösung (Tetramethylbenzidin (TMB)-Substratzübereitung gemäß Beispiel 1 von DE 35 41 979) zugegeben und bei Raumtemperatur für 30 min inkubiert;

g) Danach wurden pro Vertiefung 100 µl 1 normale Schwefelsäure zur Beendigung der enzymatischen Substratumsetzung zugegeben und die Lösungen bei 450 nm gemessen;

E. Bestimmung von HBs-Antigenspezifischen Antikörpern nach dem erfindungsgemäßen Einschritt-Verfahren

a) Pro Vertiefung einer Mikrotitrationsplatte beschichtet mit einer Lösung von 1 µg/ml HBs werden ein-heitlich 25 µl der Konjugatverdünnung 1:20 vorgelegt;

b) 100 µl der zu bestimmenden unverdünnten Serumprobe wurden zugefügt. Als Vergleich wurden 100 µl unverdünntes HBs-Antigen-Antikörperfreies Human-plasma aufgetragen (Negativ-Kontrolle) sowie serielle Verdünnungen einer Probe mit bekanntem Gehalt von Antikörpern gegen HBs-Antigen;

c) Die Testplatte wurde abgedeckt und 1 h bei 37 °C inkubiert;

d) anschließend wurde der Inhalt abgesaugt, 3mal mit Phosphat-gepufferter Kochsalzlösung, enthaltend 1 ml Tween[R] 20 ml, gewaschen und 100 µl Chromogenlösung (TMB-Substratzübereitung) zuge-geben und bei Raumtemperatur für 30 min. inkubiert;

e) Danach wurden pro Vertiefung 100 µl 1 normale Schwefelsäure zur Beendigung der enzymatischen Substratumsetzung zugegeben und die Lösungen bei 450 nm gemessen;

f) Beispiele für erhaltene Ergebnisse:

Die Proben wurden auch nach den in den Abschnitten D a) bis D g) beschriebenen Zweischrittverfahren getestet. Die Ergebnisse beider Methoden sind in Tabelle 1 zusammengefaßt.

Tabelle

Extinktionen

| | 1-Schrittverfahren<br>Testdauer 90 min | 2-Schrittverfahren<br>Testdauer 18 - 25 h |
|---|---|---|
| Stark pos. Plasma | 2.000 | 2.000 |
| Schwach pos. Plasma 4 | 0.210 | 0.295 |
| Negativ-Kontrolle | 0.021 | 0.035 |
| Plasma | 0.029 | 0.041 |

Eine Probe wurde als positiv bewertet, wenn die Extinktion über einem Wert lag, der sich aus der Extinktion der Negativ-Kontrolle plus 0.050 E ergab.

Aus den Ergebnissen für die Extinktion, die ein Maß für den Gehalt an HBs-Antigen-spezifischen Antikörpern in den humanen Proben darstellt, ist zu ersehen, daß das Einschritt-Verfahren bei der Quantifizierung über die Standards mit bekanntem Antikörpergehalt dem Zweischritt-Verfahren äquvalente Ergebnisse erbringt.

Zusätzlich zu den in Tabelle 1 aufgeführten humanen Proben wurden 500 Negativ-Seren und -Plasmen sowie 150 HBs-Antikörper-haltige Seren und Plasmen in beiden Tests übereinstimend negativ bzw. positiv gefunden. Der mit beider Verfahren ermittelte Antikörpergehalt in IU/ml stimmte hervorragend überein.

Der Nachweis von HBs-Antigen-spezifischen Antikörpern nach dem beschriebenen Einschritt-Verfahren ist demnach genauso empfindlich wie der Zweischritt-Test. Im Gegensatz zu dem mehrstufigen Testverfahren gestaltet sich jedoch der Nachweis der genannten und anderer Antikörper schneller und einfacher, wie ein Vergleich des Arbeitsaufwandes für beide Verfahren zeigt, der aus den Abschnitten D und E ersichtlich wird.

Beispiel 2

Bestimmung von Hepatitis B-core-Antigen-spezifischen Immunglobulinen

A. Herstellung des Hepatitis B-core-Antigens (HBc)

Das Hepatitis B-core-Antigen wurde wie bei Stahl et al., 1982, Proc. Nat. Acad. Sci. USA 79, 1606 - 1610 beschrieben, unter Verwendung der DNA-Technologie in Escherichia coli hergestellt.

B. Enzymmarkierung des HBc-Antigens

Die Markierung des HBc-Antigens erfolgte wie im Beispiel 1 unter B für das HBs-Antigen beschrieben. Die optimale Gebrauchsverdünnung des HBc-POD-Konjugats für den Test wurde entsprechend durch Schachbrett-Titration ermittelt.

C. Beschichtung von Microtestplatten mit HBc-Antigen

Die Beschichtung erfolgte wie im Beispiel 1 unter C für das HBs-Protein beschrieben. Allerdings enthielt die Beschichtungslösung neben dem HBc- Antigen noch 1 µg/ml Gelatine.

D. Bestimmung von HBc-Antigen-spezifischen Antikörpern (Anti-HBc) im kompetitiven Test gemäß dem Stand der Technik

Pro Vertiefung der Microtestplatte wurden 25 µl Probe und anschließend 100 µl eines Anti-HBc-POD-Konjugates pipettiert. Bei den Proben handelte es sich um serielle Verdünnungen des Anti-HBc-Standardmaterials des Paul-Ehrlich-Instituts im Konzentrationsbereich von 0 bis 5 U/ml. Die Anti-HBc-Antikörper der Probe konkurrieren in der anschließenden einstündigen Inkubation bei 37 °C im Wasserbad mit dem Anti-HBc-POD-Konjugat um die Bindestellen auf der festen Phase.

Nach dem Waschen der Näpfchen wurden 100 μl einer Lösung von Tetramethylbenzidin gemäß Beispiel 1 von DE 35 41 979, zugegeben und bei Raumtemperatur für 30 min. inkubiert.

Danach wurde die Reaktion durch das Hinzufügen von 100 μl 1 n Schwefelsäure gestoppt und die Lösung bei 450 nm gemessen.

Aufgrund des kompetitiven Prinzips ergaben Proben, die kein Anti-HBc enthalten, hohe Signale, während Proben mit Anti-HBc-Antikörpern je nach Gehalt eine Inhibition des Signals bewirkten. Die Signalhöhe war also umgekehrt proportional zur Anti-HBc-Konzentration.

Eine Probe wurde als Anti-HBc-positiv gewertet, wenn die Signalreduktion im Vergleich zur negativen Kontrolle kleiner als 50% war.

E. Bestimmung von HBc-Antigen-spezifischen Antikörpern nach dem erfindungsgemäßen Einschritt-Verfahren

Pro Vertiefung der Mikrotestplatte wurden 100 μl des HBs-POD-Konjugats vorgelegt und anschließend 25 μl Probe hinzugefügt. Bei den Proben handelte es sich um serielle Verdünnungen des Anti-HBc-Standardmaterials des Paul-Ehrlich-Instituts im Konzentrationsbereich von 0 bis 5 U/ml.

Während der anschließenden einstündigen Inkubation bei 37°C im Wasserbad bildete sich bei Anti-HBc-haltigen Proben der Sandwich-Komplex zwischen dem Festphasen-HBc, dem Anti-HBc und dem HBc-POD-Konjugat aus.

Nach dem Waschen der Näpfchen wurden 100 μl TMB-Substratzubereitung zugegeben und bei Raumtemperatur für 30 min inkubiert.

Danach wurde die Reaktion durch das Hinzufügen von 100 μl 1 n Schwefelsäure gestoppt und die Lösung bei 450 nm gemessen.

Aufgrund des Sandwich-Prinzips ergaben Proben, die kein Anti-HBc enthalten, kein Signal (bzw. nur das Hintergrundsignal), während Proben mit Anti-HBc-Antikörpern je nach Gehalt ein steigendes Signal bewirken. Die Signalhöhe war also direkt proportional zur Anti-HBc-Konzentration.

Eine Probe wurde als Anti-HBc-positiv gewertet, wenn ihre Extinktion mehr als 0.025 E über dem Signal der negativen Kontrolle lag. Die Ergebnisse, erhalten nach dem erfindungsgemäßen Verfahren, stimmten mit den Ergebnissen des kompetitiven Verfahrens überein.

**Patentansprüche**

**Patentansprüche für folgende Vertragsstaaten : AT, BE, CH, DE, FR, GB, IT, LI, LU, NL, SE**

1. Bestimmung von antigenspezifischen Antikörpern in einer Probe einer biologischen Flüssigkeit dadurch gekennzeichnet, daß

   a) ein Antigen, das für den nachzuweisenden Antikörper spezifisch ist, an einen Träger
   i) durch adsorptive Bindung
   ii) durch eine direkte kovalente Bindung, oder
   iii) durch eine kovalente, durch einen bifunktionellen Spacer vermittelte Bindung Träger-gebunden wird,
   b) dieses gebunden Antigen mit der Probe einer biologischen Flüssigkeit in Kontakt gebracht wird,
   c) gleichzeitig dasselbe Antigen wie das, welches an den Träger gebunden ist, in markierter Form in den Testansatz gegeben wird,
   d) der Testansatz eine Stunde inkubiert wird,
   e) die flüssige und die feste Phase getrennt werden,
   f) die Menge der gebundenen Markierungen bestimmt wird,
   wobei als Antigen ein oder mehrere Bestandteile von folgenden Krankheitserregern verwendet werden: Cytomegalie-Virus, Rubella, Herpes Simplex Virus, Varizella Zoster Virus, Masern und Mumps, Toxoplasma gondii, Treponemen, Hepatitis A- und B-Virus, Human Immunodeficiency Virus, Hepatitis B c- und e-Antigen, Chlamydia, Epstein-Barr-Virus, Parvovirus, Rotavirus, die Erreger der Aujeszki-Krankheit, Rinderleukose oder der Brucellose.

2. Verfahren nach Anspruch 1, dadurch gekennzeichnet, daß man als Antigen Hepatitis B-surface (HBs), -core (HBc) oder -envelope (HBe) verwendet.

3. Verfahren nach Anspruch 1, dadurch gekennzeichnet, daß man als Antigen ein oder mehrer Bestandteile oder Fragmente von HIV 1 oder HIV 2 oder von beiden verwendet.

4. Verfahren nach Anspruch 1, dadurch gekennzeichnet, daß man als Antigen einen oder mehrere Bestandteile von Hepatitis A Virus verwendet.

5. Verfahren nach Anspruch 1, dadurch gekennzeichnet, daß man als Antigen einen oder mehrere Bestandteile von Non A - Non B - Hepatitis Virus verwendet.

6. Mittel enthaltend einen
   i) durch adsorptive Bindung
   ii) durch eine direkte kovalente Bindung, oder
   iii) durch eine kovalente, durch einen bifunktionellen Spacer vermittelte Bindung
   mit Antigen beladenen Träger als Festphase und ein markiertes Antigen mit gleicher Spezifität wie das Antigen der Festphase, wobei die Menge des Antigens an der Festphase und die des markierten Antigens in einem Verfahren nach Anspruch 1 ausgewählt sind.

7. Mittel nach Anspruch 6, dadurch gekennzeichnet, daß das Antigen HBs, HBc, HBe oder ein Fragment davon ist.

8. Mittel nach Anspruch 6, dadurch gekennzeichnet, daß das Antigen ein oder mehrere Bestandteile oder Fragmente von HIV 1 und/oder von HIV 2 enthält.

9. Mittel nach Anspruch 6, dadurch gekennzeichnet, daß das Antigen einen oder mehrere Bestandteile oder Fragmente von Hepatitis A Virus enthält.

10. Mittel nach Anspruch 6, dadurch gekennzeichnet, daß das Antigen einen oder mehrere Bestandteile oder Fragmente von Non A - Non B - Hepatitis Virus enthält.

11. Verwendung eines Mittels nach Anspruch 6 in einem Verfahren zur Bestimmung von antigenspezifischen Antikörpern, in dem die Probe und das markierte Antigen mit der Festphase gleichzeitig zusammengebracht werden, anschließend die Festphase von der flüssigen Phase getrennt und die Markierung an der Festphase bestimmt wird.

**Patentansprüche für folgenden Vertragsstaat : ES**

1. Bestimmung von antigenspezifischen Antikörpern in einer Probe einer biologischen Flüssigkeit dadurch gekennzeichnet, daß
   a) ein Antigen, das für den nachzuweisenden Antikörper spezifisch ist, an einen Träger
      i) durch adsorptive Bindung
      ii) durch eine direkte kovalente Bindung, oder
      iii) durch eine kovalente, durch einen bifunktionellen Spacer vermittelte Bindung Träger-gebunden wird,
   b) dieses gebundene Antigen mit der Probe einer biologischen Flüssigkeit in Kontakt gebracht wird,
   c) gleichzeitig dasselbe Antigen wie das, welches an den Träger gebunden ist, in markierter Form in den Testansatz gegeben wird,
   d) der Testansatz eine Stunde inkubiert wird,
   e) die flüssige und die feste Phase getrennt werden,
   f) die Menge der gebundenen Markierungen bestimmt wird,
   wobei als Antigen ein oder mehrere Bestandteile von folgenden Krankheitserregern verwendet werden: Cytomegalie-Virus, Rubella, Herpes Simplex Virus, Varizella Zoster Virus, Masern und Mumps, Toxoplasma gondii, Treponemen, Hepatitis A- und B-Virus, Human Immunodeficiency Virus, Hepatitis B c- und e-Antigen, Chlamydia, Epstein-Barr-Virus, Parvovirus, Rotavirus, die Erreger der Aujeszki-Krankheit, Rinderleukose oder der Brucellose.

2. Verfahren nach Anspruch 1, dadurch gekennzeichnet, daß man als Antigen Hepatitis B-surface (HBs), -core (HBc) oder -envelope (HBe) oder Fragmente davon verwendet.

3. Verfahren nach Anspruch 1, dadurch gekennzeichnet, daß man als Antigen ein oder mehrer Bestandteile oder Fragmente von HIV 1 oder HIV 2 oder von beiden verwendet.

4. Verfahren nach Anspruch 1, dadurch gekennzeichnet, daß man als Antigen einen oder mehrere Bestandteile oder Fragmente von Hepatitis A Virus verwendet.

5. Verfahren nach Anspruch 1, dadurch gekennzeichnet, daß man als Antigen einen oder mehrere Bestandteile oder Fragmente von Non A - Non B - Hepatitis Virus verwendet.

6. Verfahren zu Herstellung eines Mittels, enthaltend einen mit Antigen beladenen Träger als Festphase und ein markiertes Antigen mit gleicher Spezifität wie das Antigen der Festphase, wobei die Menge des Antigens an der Festphase und die des markierten Antigens in einem Verfahren nach Anspruch 1 ausgewählt sind.

7. Verwendung eines Mittels nach Anspruch 6 in einem Verfahren zur Bestimmung von antigenspezifischen Antikörpern, in dem die Probe und das markierte Antigen mit der Festphase gleichzeitig zusammengebracht werden, anschließend die Festphase von der flüssigen Phase getrennt und die Markierung an der Festphase bestimmt wird.

**Claims**

**Claims for the following Contracting States: AT, BE, CH, DE, FR, GB, IT, LI, LU, NL, SE.**

1. The determination of antigen-specific antibodies in a sample of a biological fluid wherein
   a) an antigen which is specific for the antibody to be detected is carrier-bound to a carrier
      i) by adsorptive binding
      ii) by a direct covalent linkage, or
      iii) by a covalent linkage mediated by a bifunctional spacer,
   b) this bound antigen is contacted with the sample of a biological fluid,
   c) at the same time the same antigen as that bound to the carrier is acded in in labeled form to the assay mixture,
   d) the assay mixture is incubated for one hour,
   e) the liquid and the solid phase are separated,
   f) the amount of the bound labeling is determined,
   wherein theantigen used is one or more parts of the following pathogens: cytomegalovirus, rubella, herpes simplex virus, varicella zoster virus, measles and mumps, Toxoplasma gondii, treponemas, hepatitis A and B virus, human immunodeficiency virus, hepatitis Bc and e antigen, chlamydia, Epstein-Barr virus, parvovirus, rotavirus, the agents causing Aujeszki disease, bovine leukosis or brucellosis.

2. The method as claimed in claim 1, wherein hepatitis B surface (HBs), core (HBc) or envelope (HBe) antigen is used.

3. The method as claimed in claim 1, wherein one or more constituents or fragments of HIV 1 or HIV 2 or of both are used as antigen.

4. The method as claimed in claim 1, wherein one or more constitutents of hepatitis A virus are used as antigen.

5. The method as claimed in claim 1, wherein one or more constituents of non-A, non-B hepatitis virus are used as antigen.

6. A composition containing a carrier loaded with antigen
      i) by adsorptive binding
      ii) by a direct covalent linkage, or
      iii) by a covalent linkage mediated by a bifunctional spacer,
   as solid phase and a labeled antigen with the same specificity as the antigen on the solid phase, where the amount of the antigen on the solid phase and that of the labeled antigen are selected in a method as claimed in claim 1.

7. A composition as claimed in claim 6, wherein the antigen is HBs, HBc, HBe or a fragment thereof.

8. A composition as claimed in claim 6, wherein the antigen contains one or more constituents or fragments of HIV 1 and/or of HIV 2.

9. A composition as claimed in claim 6, wherein the antigen contains one or more constituents or fragments of hepatitis A virus.

10. A composition as claimed in claim 6, wherein the antigen contains one or more constituents or fragments of non-A, non-B hepatitus virus.

11. The use of a composition as claimed in claim 6 in a method for determining antigen-specific antibodies, in which the sample and the labeled antigen are simultaneously contacted with the solid phase, subsequently the solid phase is separated from the liquid phase, and the labeling on the solid phase is determined.

**Claims for the following Contracting State : ES**

1. The determination of antigen-specific antibodies in a sample of a biological fluid wherein
   a) an antigen which is specific for the antibody to be detected is carrier-bound to a carrier
      i) by adsorptive binding
      ii) by a direct covalent linkage, or
      iii) by a covalent linkage mediated by a bifunctional spacer,
   b) this bound antigen is contacted with the sample of a biological fluid,
   c) at the same time the same antigen as that bound to the carrier is added in in labeled form to the assay mixture,
   d) the assay mixture is incubated for one hour,
   e) the liquid and the solid phase are separated,
   f) the amount of the bound labeling is determined,
   wherein the antigen used is one or more parts of the following pathogens: cytomegalovirus, rubella, herpes simplex virus, varicella zoster virus, measles and mumps, Toxoplasma gondii, treponemas, hepatitis A and B virus, human immunodeficiency virus, hepatitis Bc and e antigen, chlamydia, Epstein-Barr virus, parvovirus, rotavirus, the agents causing Aujeszki disease, bovine leukosis or brucellosis.

2. The method as claimed in claim 1, wherein hepatitis B surface (HBs), core (HBc) or envelope (HBe) antigen or fragments thereof are used.

3. The method as claimed in claim 1, wherein one or more constituents or fragments of HIV 1 or HIV 2 or of both are used as antigen.

4. The method as claimed in claim 1, wherein one or more constitutents or fragments of hepatitis A virus are used as antigen.

5. The method as claimed in claim 1, wherein one or more constituents or fragments of non-A, non-B hepatitis virus are used as antigen.

6. A process for preparing a composition containing a carrier loaded with antigen as solid phase and a labeled antigen with the same specificity as the antigen on the solid phase, where the amount of the antigen on the solid phase and that of the labeled antigen are selected in a method as claimed in claim 1.

7. The use of a composition as claimed in claim 6 in a method for determining antigen-specific antibodies, in which the sample and the labeled antigen are simultaneously contacted with the solid phase, subsequently the solid phase is separated from the liquid phase, and the labeling on the solid phase is determined.

## Revendications

### Revendications pour les Etats contractants suivants : AT, BE, CH, DE, FR, GB, IT, LI, LU, NL, SE

1. Détermination d'anticorps spécifiques d'antigènes dans un échantillon d'un liquide biologique, caractérisée en ce que
   a) un antigène qui est spécifique de l'anticorps à détecter est fixé sur un support
      I) par liaison par adsorption,
      II) par une liaison covalente directe ou
      III) par une liaison covalente médiée par un espaceur bifonctionnel,
   b) cet antigène fixé est mis en contact avec l'échantillon d'un liquide biologique,
   c) en même temps le même antigène que celui qui est fixé sur le support est introduit, sous forme marquée, dans le mélange d'essai,
   d) on met le mélange d'essai à incuber pendant 1 heure,
   e) on sépare la phase liquide et la phase solide,
   f) on détermine la quantité des marquages fixés,
   avec utilisation, en tant qu'antigène, d'un ou plusieurs composants des agents pathogènes suivants: cytomégalovirus, virus de la rubéole, virus *Herpes simplex,* virus varicelle-zona, virus de la rougeole et des oreillons, *Toxoplasma gondii*, tréponèmes, virus de l'hépatite A et de l'hépatite B, virus de l'immunodéficience humaine, antigène c et e de l'hépatite B, *Chlamydia*, virus Epstein-Barr, parvovirus, rotavirus, les agents pathogènes de la maladie d'Aujeski, de la leucose bovine et de la brucellose.

2. Procédé selon la revendication 1, caractérisé en ce que, comme antigène, on utilise l'antigène de surface (HBs), de nucléocapside (HBc) ou d'enveloppe (HBe) de l'hépatite B.

3. Procédé selon la revendication 1, caractérisé en ce que, comme antigène, on utilise un ou plusieurs composants ou fragments du HIV 1 ou du HIV 2, ou des deux.

4. Procédé selon la revendication 1, caractérisé en ce que, comme antigène, on utilise un ou plusieurs composants du virus de l'hépatite A.

5. Procédé selon la revendication 1, caractérisé en ce que, comme antigène, on utilise un ou plusieurs composants du virus de l'hépatite non A - non B.

6. Agent contenant, en tant que phase solide, un support chargé d'antigène
      I) par liaison par adsorption,
      II) par une liaison covalente directe ou
      III) par une liaison covalente médiée par un espaceur bifonctionnel,
   et un antigène marqué ayant la même spécificité que l'antigène de la phase solide, la quantité de l'antigène sur la phase solide et celle de l'antigène marqué étant choisies dans un procédé selon la revendication 1.

7. Agent selon la revendication 6, caractérisé en ce que l'antigène est HBs, HBc, HBe ou un fragment de ceux-ci.

8. Agent selon la revendication 6, caractérisé en ce que l'antigène contient un ou plusieurs composants ou fragments du HIV 1 et/ou du HIV 2.

9. Agent selon la revendication 6, caractérisé en ce que l'antigène contient un ou plusieurs composants ou fragments du virus de l'hépatite A.

10. Agent selon la revendication 6, caractérisé en ce que l'antigène contient un ou plusieurs composants ou fragments du virus de l'hépatite non A - non B.

11. Utilisation d'un agent selon la revendication 6, dans un procédé pour la détermination d'anticorps spécifiques d'antigènes, dans lequel on met simultanément l'échantillon et l'antigène marqué en contact avec la phase solide, on sépare ensuite la phase solide de la phase liquide et on détermine le marquage sur la phase solide.

**Revendications pour l'Etat contractant suivant : ES**

1. Détermination d'anticorps spécifiques d'antigènes dans un échantillon d'un liquide biologique, caractérisée en ce que

    a) un antigène qui est spécifique de l'anticorps à détecter est fixé sur un support

        I) par liaison par adsorption,

        II) par une liaison covalente directe ou

        III) par une liaison covalente médiée par un espaceur bifonctionnel,

    b) cet antigène fixé est mis en contact avec l'échantillon d'un liquide biologique,

    c) en même temps le même antigène que celui qui est fixé sur le support est introduit, sous forme marquée, dans le mélange d'essai,

    d) on met le mélange d'essai à incuber pendant 1 heure,

    e) on sépare la phase liquide et la phase solide,

    f) on détermine la quantité des marquages fixés,

avec utilisation, en tant qu'antigène, d'un ou plusieurs composants des agents pathogènes suivants: cytomégalovirus, virus de la rubéole, virus *Herpes simplex*, virus varicelle-zona, virus de la rougeole et des oreillons, *Toxoplasma gondii*, tréponèmes, virus de l'hépatite A et de l'hépatite B, virus de l'immunodéficience humaine, antigène c et e de l'hépatite B, *Chlamydia*, virus Epstein-Barr, parvovirus, rotavirus, les agents pathogènes de la maladie d'Aujeski, de la leucose bovine et de la brucellose.

2. Procédé selon la revendication 1, caractérisé en ce que, comme antigène, on utilise l'antigène de surface (HBs), de nucléocapside (HBc) ou d'enveloppe (HBe) de l'hépatite B.

3. Procédé selon la revendication 1, caractérisé en ce que, comme antigène, on utilise un ou plusieurs composants ou fragments du HIV 1 ou du HIV 2, ou des deux.

4. Procédé selon la revendication 1, caractérisé en ce que, comme antigène, on utilise un ou plusieurs composants ou fragments du virus de l'hépatite A.

5. Procédé selon la revendication 1, caractérisé en ce que, comme antigène, on utilise un ou plusieurs composants ou fragments du virus de l'hépatite non A - non B.

6. Procédé pour la préparation d'un agent contenant, en tant que phase solide, un support chargé d'antigène, et un antigène marqué ayant la même spécificité que l'antigène de la phase solide, la quantité de l'antigène sur la phase solide et celle de l'antigène marqué étant choisies dans un procédé selon la revendication 1.

7. Utilisation d'un agent selon la revendication 6, dans un procédé pour la détermination d'anticorps spécifiques d'antigènes, dans lequel on met simultanément l'échantillon et l'antigène marqué en contact avec la phase solide, on sépare ensuite la phase solide de la phase liquide et on détermine le marquage sur la phase solide.